# EUROPÄISCHE PATENTSCHRIFT

(11) **EP 2 408 461 B1**
(45) Veröffentlichungstag und Bekanntmachung des Hinweises auf die Patenterteilung: **27.01.2016**
(21) Anmeldenummer: 10710799.7
(22) Anmeldetag: 17.03.2010
(51) Int. Cl.: A61K 36/324

(54) **VERFAHREN ZUR EXTRAKTION VON PFLANZLICHEN HARZEN SOWIE DADURCH GEWONNENE EXTRAKTIONSPRODUKTE UND DEREN VERWENDUNG**
METHOD FOR EXTRACTING PLANT RESINS, EXTRACTION PRODUCTS OBTAINED THEREBY AND USE OF SAME
PROCÉDÉ D'EXTRACTION DE RÉSINES VÉGÉTALES, AINSI QUE PRODUITS D'EXTRACTION ANSI OBTENUS ET LEUR UTILISATION

(30) Priorität: 17.03.2009 DE 102009013553
(43) Veröffentlichungstag der Anmeldung: 25.01.2012
(73) Patentinhaber: Ertelt, Herta, 72406 Bisingen (DE)
(72) Erfinder: ERTELT, Winfried, 72406 Bisingen (DE)
(74) Vertreter: Hiebl, Inge Elisabeth
(86) Internationale Anmeldenummer: PCT/EP2010/001688
(87) Internationale Veröffentlichungsnummer: WO 2010/105821

(56) Entgegenhaltungen:
- US-A1- 2005 192 251

## Beschreibung

Die Erfindung betrifft ein Verfahren zur Extraktion von pflanzlichen Harzen, die pharmakologisch aktive Inhaltsstoffe enthalten, insbesondere Weihrauchharz, unter Verwendung eines hydrophilen Lösungsmittelgemisches in Kombination mit einem oder mehreren Tensiden. Die Erfindung betrifft insbesondere ein Verfahren zur gleichzeitigen Extraktion von hydrophilen und lipophilen Inhaltsstoffen mit pharmakologischer Wirkung aus pflanzlichen Harzen, die pharmakologisch aktive Inhaltsstoffe enthalten.

Pflanzliche Harze enthalten häufig pharmakologisch aktive Inhaltsstoffe, die zur Anwendung am oder im menschlichen oder tierischen Organismus geeignet sind. Beispiel für solche Harze sind Boswelliaharze, Mastix, Kolophonium, Dammar, Myrrhe, Elemi, Copal, Terpentin, Sandarak, Balsam, Gummilack. Um die pharmakologisch aktiven Inhaltsstoffe einer therapeutischen Verwendung zuführen zu können, müssen diese aus dem Harz in geeigneter Weise isoliert werden. Insbesondere für Weihrauchharze bzw. Boswelliaharze bzw. einen daraus enthaltenen Extrakt sind mehrere medizinische Anwendungen beschrieben.

Die WO 90/01937 beschreibt, dass α-Bowelliasäureacetat und β-Boswelliasäureacetat und deren Analoga Topoisomerase-I und Topoisomerase-II hemmen können. Daher schlägt diese Druckschrift vor, die genannten Verbindungen zur Behandlung von verschiedenen Krebsarten einzusetzen.

Die DE 42 01 904 A und die dazu äquivalente EP 552 657 A offenbaren, dass reine Boswelliasäure, physiologisch annehmbare Salze davon, Derivate davon und Salze der Derivate oder eine Boswelliasäure-enthaltende pflanzliche Zubereitung Entzündungsvorgänge bekämpfen können, die durch gesteigerte Leukotrienbildung hervorgerufen werden.

Die DE-44 44 288 A offenbart, dass Weihrauch, Weihrauchextrakte, in Weihrauch enthaltene Substanzen, Boswelliasäure sowie Derivate und Salze dieser Verbindungen bei der Behandlung der Alzheimer Erkrankung erfolgreich eingesetzt werden können.

Die DE 44 45 728 A hingegen verwendet reine Boswelliasäure, physiologisch annehmbare Salze davon, Derivate davon oder Salze der Derivate sowie pflanzliche Zubereitungen, die eine Boswelliasäure enthalten, zur Behandlung von Hirntumoren.

Die US 2005/192251 beschreibt ein Verfahren zur Gewinnung einer wasserlöslichen, bioaktiven Fraktion aus dem Harzexsudat von Boswellia serrata umfassend die Extraktion des Harzexsudats mit Ethanol oder Methanol, wodurch das Mark erhalten wird, welches dann mit Wasser extrahiert und mit Ethanol gefällt wird. Gemäß diesem Verfahren wird die Polysaccharidfraktion des Harzes von Boswellia serrata erhalten.

Weihrauchextrakt enthaltende pharmazeutische Zusammensetzungen sind bereits im Stand der Technik bekannt und auch kommerziell erhältlich, beispielsweise von der Firma Gufic, Indien, unter der Bezeichnung Sallaki^{®} Gufic (ehemals H15^{®} Gufic). Hierbei handelt es sich um Tabletten, die als Wirkstoff einen durch lipophile Extraktion gewonnenen Trockenextrakt aus Olibanum des Boswellia serrata enthalten.

Ausweislich der DE 44 44 288 A enthält eine Tablette 400 mg Trockenextrakt aus Olibanum, wobei als Extraktionsmedium ein Chloroform/Methanol-Gemisch eingesetzt wurde. Darüber hinaus sind im Stand der Technik Extrakte aus Olibanum bekannt, die mittels ethanolischer Extraktion gewonnen wurden.

Im Stand der Technik sind jedoch keine zufriedenstellenden Verfahren zur Extraktion von pflanzlichen Harzen und insbesondere zur Extraktion von Weihrauchharzen bekannt. Insbesondere sind keine Verfahren bekannt, die die Isolierung von pharmakologisch aktiven Inhaltsstoffen in hoher Reinheit für eine medizinische Verwendung erlauben.

Der vorliegenden Erfindung liegt somit die Aufgabe zugrunde, ein Verfahren zur Extraktion von pflanzlichen Harzen und insbesondere von Boswelliaharzen bereitzustellen, mit dem die im Harz enthaltenen pharmakologisch wirksamen Inhaltsstoffe schonend und unter Erhalt der pharmakologischen Wirksamkeit extrahiert werden können. Das erfindungsgemäße Verfahren soll die pharmakologisch wirksamen Inhaltsstoffe eines Harzes in hoher Reinheit und mit hoher Ausbeute ergeben. Das Verfahren soll einfach, ökologisch, ökonomisch, sicher und reproduzierbar sein und sowohl im kleinen als auch großtechnischen Maßstab durchführbar sein. Das Verfahren soll das natürliche Spektrum pharmakologisch relevanter Inhaltsstoffe eines Harzes abbilden können, unabhängig von deren Lipophilie bzw. Polarität.

Erfindungsgemäß wird diese Aufgabe gelöst durch ein Verfahren zur Extraktion von pflanzlichen Harzen, die pharmakologisch aktive Inhaltsstoffe enthalten, dadurch gekennzeichnet, dass man a) ein pflanzliches Rohharz aus einer Harz enthaltenden Pflanze bereitstellt, b) das Rohharz mit einem Extraktionsmedium umfassend einen Wasseranteil von 50-90 Gew.-%, ein oder mehrere hydrophile Lösungsmittel und ein oder mehrere Tensid(e) extrahiert, und c) das so erhaltene Extraktionsprodukt in an sich bekannter Weise isoliert und gegebenenfalls weiter verarbeitet.

Die Erfindung betrifft ferner das durch dieses Verfahren erhaltene Extraktionsprodukt bzw. das Extraktionsprodukt zur Verwendung als Arzneimittel, Medizinprodukt, funktionelles Lebensmittel, Nahrungsergänzungsmittel oder Kosmetikum.

Das erfindungsgemäße Verfahren eignet sich grundsätzlich zur Extraktion von pflanzlichen Harzen, die pharmakologisch aktive Inhaltsstoffe enthalten, wie vorstehend aufgeführt wurde. Bevorzugt eignet sich das erfindungsgemäße Verfahren zur Extraktion von Weihrauchharz bzw. Boswelliaharzen. Bevorzugt sind die Boswelliaharze afrikanischer und/oder arabischer sowie indischer Boswelliaharze. Am meisten bevorzugt ist das Harz von Boswellia papyrifera.

Mit dem erfindungsgemäßen Verfahren können sowohl Harze einzelner Boswelliaspezies als auch Mischungen verschiedener Boswelliaspezies extrahiert werden, zum Beispiel Boswellia papyrifera mit Boswellia serrata. Natürlich können auch Harze anderer Vertreter der Boswelliafamilie, wie zum Beispiel Boswellia sacra, Boswellia carterii, Boswellia frereana, Boswellia serrata eingesetzt werden.

Überraschenderweise wurde gefunden, dass durch Extraktion eines pflanzlichen Harzes und insbesondere Weihrauch mit einem hydrophilen Extraktionsmedium umfassend einen Wasseranteil von 50-90 Gew.-%, einen oder mehrere ein- oder mehrwertige Alkohole und Tenside mit O/W-Charakter, ein Harzextrakt und insbesondere ein Weihrauchextrakt erhalten wird, der eine hohe pharmakologische Wirksamkeit aufweist und somit gezielt einer therapeutischen Verwendung zugeführt werden kann.

Erfindungsgemäß wird in einem ersten Schritt ein pflanzliches Rohharz aus einer Harz enthaltenden Pflanze, bevorzugt Weihrauch, bereitgestellt. Das Harz kann in handelstypischen Körnungsgrößen (Granen, Erbsen, aber auch fein oder feinst vermahlen) als Ausgangsmaterial eingesetzt werden. Die Vermahlung des Harzes erfolgt durch gängige Methoden der Pharmazie oder Technologie, die einem Fachmann auf dem Gebiet bekannt sind. Das Rohharz wird mit dem Extraktionsmedium, umfassend ein Gemisch aus einem Wasseranteil von 50-90 Gew.-%, einem oder mehreren hydrophilen Lösungsmittel(n) und einem oder mehreren Tensid(en), extrahiert.

Gemäß einer bevorzugten Ausführungsform der Erfindung wird das Harz zu Beginn unter Rühren mittels üblichen Methoden (Rührer) in Bewegung gehalten, um ein vollständiges Benetzen der Harzkörner mit dem Extraktionsmedium zu gewährleisten.

Sobald das Harz unter dem Einfluss des Lösungsmittelgemisches Auflösungstendenzen zeigt bzw. deutlich aufgeweicht vorliegt, wird abermals gründlich durchgerührt und die Lösung für o. g. Zeitraum stehen gelassen.

So wird gewährleistet, dass im Extraktionsmedium unlösliche Komponenten (Matrixbestandteile des Harzes wie Zucker, bestimmte Schleimstoffe, etc.) - teilweise auch nach Koagulation - sedimentieren und abgetrennt werden können.

Eine weitere Ausführungsform der vorliegenden Erfindung ist die kontinuierliche Extraktion durch Abtrennen des Extraktes und Zuführung frischen Extraktionsmediums. Dies kann mit den bekannten Methoden der pharmazeutischen Technologie oder anderen Verfahren erfolgen.

Bevorzugt erfolgt der Extraktionsvorgang bei Raumtemperatur, d.h. in einem Temperaturbereich zwischen 18°C und 25°C, besonders bevorzugt bei 25°C, um thermische Belastung sowie Zerstörung der genuinen Wirkstoffe zu vermeiden. In Abhängigkeit von dem zu extrahierenden Harz und den darin enthaltenen Inhaltsstoffen kann das Extraktionsverfahren grundsätzlich bei Temperaturen zwischen 0°C und 99°C durchgeführt werden. Durch die schonende Durchführung des Extraktionsverfahrens wird gewährleistet, dass die Extraktion schonend, kostengünstig, energiesparend und damit umweltfreundlich sowie (groß-)technisch leicht umsetzbar ist (im Gegensatz zu der thermisch und energetisch aufwendigen Soxhletextraktion im Stand der Technik).

Der Extraktionsschritt erfolgt im Allgemeinen so lange bis sämtliche pharmakologisch aktiven Inhaltsstoffe des Harzes extrahiert sind. Bevorzugt ist die Extraktionszeit größer 1 h, kleiner 48 h, mehr bevorzugt größer 6 h und kleiner 36 h, noch mehr bevorzugt größer 12 h und kleiner 30 h, am meisten bevorzugt größer 20 h und kleiner 26 h.

So hat sich gezeigt, dass diverse Quellungs- und Diffusions- bzw. Lösungsvorgänge nach ca. 24 h ein Maximum erreicht haben.

Das erfindungsgemäß verwendete Extraktionsmedium enthält ein Gemisch aus einem Wasseranteil von 50-90 Gew.-%, einem oder mehreren hydrophilen Lösungsmittel(n) und einem oder mehreren Tensid(en) oder besteht daraus. Charakteristisch für das erfindungsgemäße Verfahren ist somit die Kombination verschiedener Lösungsmittel unterschiedlicher, d.h. abgestufter, Polarität, wodurch die Extraktion eines breiten Spektrums verschiedener Inhaltsstoffe mit pharmakologischer Aktivität ermöglicht wird.

Das hydrophile Lösungsmittel umfasst bevorzugt ein- oder mehrwertige Alkohole niedriger Kettenlänge. In Kombination mit Wasser werden dadurch die polaren Wirkstoffe aus Harzen (z.B. Neutralbestandteile, ätherische Öle, saponile Schleimstoffe etc.) erhalten.

Dabei üben die niederkettigen Alkohole in Verbindung mit Wasser einen quellenden Effekt auf das Harz bzw. die Harzkörner aus und dienen als Weichmacher bzw. Lösungsmittel für Harze.

Geeignete mehrwertige Alkohole weisen eine Kettenlänge von C₂-C₈, (C₂, C₃, C₄, C₅, C₆, C₇, C₈) auf. Bevorzugt sind die Kettenlängen C₂, C₃, C₄, C₅, C₆ und besonders bevorzugt C₂, C₃, C₄.

Geeignete mehrwertige Alkohole sind Glycerin, Ethylenglykol, Propylenglykol, Butylenglykol, Pentylenglykol.

Lipophile Wirkstoffe (z.B. Harzsäuren) aus demselben Harz können in einem Schritt, d.h. gleichzeitig, mittels einer geeigneten Konzentration niederkettiger, einwertiger Alkohole herausgelöst werden und durch die Anwesenheit eines geeigneten, vorzugsweise O/W-Tensids oder durch Mischungen verschiedener Tenside in Lösung gehalten werden.

Erfindungsgemäß wird durch die Anwesenheit eines Tensids oder der Kombination verschiedener Tenside eine verbesserte Benetzung des Harzes ermöglicht. Weiterhin wird durch TensidZusatz der Phasentransfer für lipophile Wirkstoffe katalysiert.

Geeignete niederkettige, einwertige Alkohole können eine Kettenlänge von C₁-C₁₀ (C₁, C₂, C₃, C₄, C₅, C₆, C₇, C₈, C₉, C₁₀) aufweisen, bevorzugt kleiner/gleich C₈ (C₁, C₂, C₃, C₄, C₅, C₆, C₇, C₈), besonders bevorzugt kleiner/gleich C₆ (C₁, C₂, C₃, C₄, C₅, C₆), am meisten bevorzugt kleiner/gleich C₄ (C₁, C₂, C₃, C₄). Noch mehr bevorzugt kleiner/gleich C₃ (C₁, C₂, C₃).

Bevorzugt sind die einwertigen Alkohole Methanol und Ethanol.

Erfindungsgemäß bevorzugt ist eine Kombination aus Glycerin, Propylenglykol und Ethanol und somit ein wässriges Extraktionsmedium umfassend oder enthaltend Glycerin, Propylenglykol, Ethanol und ein Netzmittel.

Geeignete O/W-Tenside bzw. Tensid-Kombinationen sind alle Stoffe, die in der Lage sind in hydrophiler Umgebung alleine oder in Kombination, lipophile, wasserunlösliche Stoffe zu solubilisieren. Bevorzugt haben die erfindungsgemäßen Tenside einen hohen HLB-Wert.

Hierzu zählen vor allem alle in der Pharmazie, Medizin, Lebensmittelchemie und Kosmetik zugelassenen Tenside wie zum Beispiel Polysorbate, Säureester, Zuckertenside, Polyoxyethylene, Macrogolstearate, emulgierende Cetylstearylalkohole.

Erfindungsgemäß können auch andere löslichkeitsvermittelnde Stoffe, die auf Pickering-Effekten beruhen (z.B. Hydroxyethylcellulose, Titandioxid, u.a.) verwendet werden.

Bevorzugt sind erfindungsgemäß Tenside bzw. Tensid-Kombinationen aus der Gruppe der Polysorbate. Besonders bevorzugt sind Polysorbate 60 und 80. Am meisten bevorzugt Polysorbat 80.

Erfindungsgemäß besonders bevorzugt ist ein Extraktionsmedium umfassend Glycerin, Propylenglykol, Ethanol, Polysorbat 80 und Wasser.

Entscheidend für den Erhalt des pharmakologisch aktiven Extraktes durch das erfindungsgemäße Extraktionsverfahren ist das optimale Verhältnis der beschriebenen LösungsmittelKomponenten zueinander und den gewählten Extraktionsparametern.

Bevorzugt ist es, wenn der einwertige Alkohol und insbesondere Ethanol in dem Extraktionsmedium mit einem Anteil von 0,5 bis 50 Gew.-% enthalten ist. Dadurch wird sichergestellt, dass die in Ethanol löslichen Wirkstoffe des Harzes, bevorzugt des Weihrauchs, weitgehend vollständig extrahiert werden können. Vorzugsweise ist der Anteil an Ethanol in dem Extraktionsmedium 1 bis 40 Gew.-%, bevorzugt 1,5 bis 20 Gew.-%, mehr bevorzugt 2 bis 10 Gew.-% und am meisten bevorzugt 3 bis 6 Gew.-% bezogen auf das Gesamtgewicht des Extraktionsmediums.

Entsprechend einer weiter bevorzugten Ausführungsform der Erfindung ist der mehrwertige Alkohol und insbesondere Glycerin in dem Extraktionsmedium in einem Anteil von 1 bis 20 Gew.-% enthalten. Dadurch wird gewährleistet, dass die pharmakologisch aktiven Substanzen des Harzes, bevorzugt des Weihrauchs sowie Hilfsstoffe überhaupt und nahezu vollständig dem Rohharz entzogen werden. Vorzugsweise ist Glycerin in dem Extraktionsmedium mit einem Anteil von 2 bis 15 Gew.-% enthalten, bevorzugt von 3 bis 12 Gew.-%, mehr bevorzugt von 4 bis 10 Gew.-% und am meisten bevorzugt von 5 bis 9 Gew.-% bezogen auf das Gesamtgewicht des Extraktionsmediums.

Weiter bevorzugt ist es, wenn Propylenglykol in dem Extraktionsmedium mit einem Anteil von 0,3 bis 10 Gew.-% enthalten ist. Dadurch wird gewährleistet, dass das Harz und insbesondere der Weihrauch so weit aufgelöst wird bzw. so weit aufquillt, dass die Wirkstoffe des Weihrauchs sowie pharmazeutische Hilfsstoffe weitgehend vollständig aus dem Rohharz extrahiert werden können. Vorzugsweise ist Propylenglykol in dem Extraktionsmedium mit einem Anteil von 0,5 bis 9 Gew.-% enthalten, bevorzugt von 1 bis 6 Gew.-%, mehr bevorzugt von 1,3 bis 5 Gew.-% und am meisten bevorzugt von 1,7 bis 3,5 Gew.-% bezogen auf das Gesamtgewicht des Extraktionsmediums.

Entsprechend einer besonders bevorzugten Ausführungsform der Erfindung ist das Tensid (Netzmittel) Polysorbat 80. Es wurde festgestellt, dass insbesondere Polysorbat 80 in der Lage ist, Wirkstoffe mit verhältnismäßig großem lipophilen Charakter dem Harz, bevorzugt dem Weihrauch, zu entziehen.

Gemäß einer weiteren bevorzugten Ausführungsform der Erfindung ist das Netzmittel in dem Extraktionsmedium mit einem Anteil von 0,01 bis 3 Gew.-% enthalten. Unterhalb von 0,01 Gew.-% ist ein Auszug von lipophilen oder überwiegend lipophilen pharmakologisch wirksamen Bestandteilen des Harzes nur unter erschwerten Bedingungen möglich, während oberhalb eines Anteils von 3 Gew.-% kein verbessertes Extraktionsverhalten des Extraktionsmediums bzgl. der genannten Substanzen beobachtet werden kann. Vorzugsweise ist das Netzmittel in dem Extraktionsmedium mit einem Anteil von 0,03 bis 2,0 Gew.-% enthalten, mehr bevorzugt von 0,05 bis 1,0 Gew.-% und am meisten bevorzugt von 0,08 bis 0,15 Gew.-% bezogen auf das Gesamtgewicht des Extraktionsmediums.

Überraschenderweise wurde gefunden, dass trotz eines Wasseranteils von größer 50 Gew.-% lipophile Wirkstoffe aus Harzen, bevorzugt Boswelliasäuren, gewonnen werden können.

Um eine möglichst vollständige Extraktion des Harzes, insbesondere des Weihrauchs, zu erhalten, ist bei der Extraktion das Gewichtsverhältnis von Harz/Weihrauch zu Extraktionsmedium größer als 1:10, vorzugsweise größer als 1:8, bevorzugt größer als 1:6, mehr bevorzugt größer als 1:4 und am meisten bevorzugt größer als 1:3.

Gemäß einer weiteren bevorzugten Ausführungsform der Erfindung wird der pH-Wert des erhaltenen Extrakts auf einen Bereich zwischen 4 und 8, bevorzugt zwischen 4,5 und 7,8, noch bevorzugter zwischen 5,0 und 7,6 eingestellt. Am bevorzugtesten wird der pH-Wert auf einen Bereich zwischen 5,5 und 7,4 eingestellt. Die Einstellung des pH-Werts erfolgt mit physiologisch verträglichen Pufferlösungen. Somit ist eine Verwendung des Harzes in physiologischen pH-Bereichen gegeben.

Wie beispielhaft an der Extraktion eines Weihrauchharzes gezeigt, liefert das erfindungsgemäße Verfahren einen Extrakt, der durch die spezifische Zusammensetzung verschiedener pharmakologisch aktiver Inhaltsstoffe namentlich Boswelliasäuren und Neutralbestandteile gekennzeichnet ist. Das erfindungsgemäße Verfahren ist den bisher zur Gewinnung von Boswelliaextrakten verwendeten Verfahren klar überlegen, da die bisherigen Verfahren hauptsächlich die alpha-keto-Boswelliasäure sowie die keta-Boswelliasäure angereichert haben. Überraschenderweise wurde gefunden, dass erfindungsgemäß durch Extraktion von Weihrauch mittels eines hydrophilen Extraktionsmediums, enthaltend Wasser, Alkohole, Polyole und Tenside mit vorzugsweise O/W-Charakter, ein Weihrauchextrakt erhalten wird, der eine hohe pharmakologische Wirksamkeit aufweist. Damit eignet sich der mit dem erfindungsgemäßen Verfahren erhaltene Extrakt zur gezielten Behandlung von Krankheiten, die durch inflammatorische Prozesse gekennzeichnet sind, wie beispielsweise Erkrankungen des rheumatischen Formenkreises, Erkrankungen des Bewegungsapparates, entzündlicher Haut- und Darmerkrankungen, schmerz-assoziierte Erkrankungen oder Tumorerkrankungen.

Die Erfindung betrifft somit auch eine therapeutische Zusammensetzung, umfassend einen gemäß dem erfindungsgemäßen Verfahren erhaltenen Weihrauchextrakt zur Verwendung zur Behandlung von Krankheiten des rheumatischen Formenkreises, insbesondere zur Behandlung von degenerativen Gelenk- und Wirbelsäulenerkrankungen, beispielsweise Arthrose, von entzündlichen rheumatischen Erkrankungen, beispielsweise rheumatoide Arthritis, Morbus Bechterew, systemische Lupus erythematodes, von Stoffwechselerkrankungen mit rheumatischen Beschwerden, zum Beispiel Gicht, oder von Weichteilrheumatismus, zum Beispiel Fibromyalgie.

Erfindungsgemäß können die therapeutischen Zusammensetzungen bzw. Arzneimittel in an sich bekannter Art und Weise für übliche Verabreichungswege formuliert werden. Hier sind topische, orale, parenterale, rektale und intranasale Verabreichungen bevorzugt. Es ist auch möglich, dass die Formulierung für die Inhalation oder Insufflation ausgestaltet ist. Auch bevorzugt sind Arzneimittel für die intrakraniale oder intrathekale Verabreichung.

Flüssige Zubereitungen für die orale Verabreichung können beispielsweise als wässrige oder ölige Lösungen, Sirupe, Elixiere, Emulsionen oder Suspensionen vorliegen. Es ist auch möglich, dass die Formulierungen als Trockenprodukt für die Rekonstitution mit einem geeigneten Lösungsmittel, insbesondere Wasser, vorliegen.

Gemäß einer besonders bevorzugten Ausführungsform der erfindungsgemäßen Verwendung ist das Arzneimittel oder das Kosmetikum eine topisch zu verabreichende Salbe. Es wurde festgestellt, dass der durch das erfindungsgemäße Verfahren erhältliche Extrakt sehr gut durch die Haut penetrieren kann, so dass im Bereich der Auftragungsstelle im Körpergewebe sehr hohe Konzentrationen an Wirkstoffen erzielt werden können. Topisch zu verabreichende Salben sind daher insbesondere zur Behandlung von Krankheiten des rheumatischen Formenkreises zu empfehlen und dementsprechend erfindungsgemäß bevorzugt.

Alternativ zur topisch zu verabreichenden Salbe handelt es sich bei dem Arzneimittel vorzugsweise um ein topisch zu verabreichendes Gel. Beispielsweise die Kombination eines Hydrogels, welches normalerweise austrocknend wirkt, mit lipohilen und feuchtigkeitsspendenden Bestandteilen ermöglicht es, dass die Wirkstoffe schnell in die Haut einziehen ohne einen Fettfilm zu hinterlassen.

Der durch dieses Extraktionsverfahren erhaltene Extrakt ist dadurch gekennzeichnet, dass er einfach, ökologisch, ökonomisch und schnell hergestellt werden kann, ohne auf die bekannten in der Literatur beschriebenen rein lipophilen Lösungsmittel, die meist ein toxisches Potential (vgl. Extraktionen mit Methanol und/oder Dichlormethan) aufweisen, zurückzugreifen.

Der erfindungsgemäß erhältliche Weihrauchextrakt und insbesondere die darin enthaltenen Boswelliasäuren bzw. deren Derivate oder deren Salze sowie andere darin enthaltene pharmakologisch aktive Stoffe, die nicht den Boswelliasäuren zuzuordnen sind, weisen eine sehr geringe Toxizität auf und sind in der Regel sehr gut verträglich.

Das erfindungsgemäße Extraktionsverfahren ist weiterhin dadurch gekennzeichnet, dass es Extrakte mit reproduzierbarer Qualität liefert (vgl. Beispiele).

Ein weiteres Kennzeichen der vorliegenden Erfindung ist der Verzicht auf energieintensive Methoden wie es bei Extraktionen mittels Soxhlet-Apparaturen, bei Partialsynthesen oder Derivatisierungen von Boswelliasäuren der Fall ist. Die Extraktion kann unter geringem Energieeinsatz, der für die mechanische Bewegung des Extraktionsgutes notwendig ist, bei Raumtemperatur durchgeführt werden.

Dabei wurde überraschenderweise festgestellt, dass durch die Gesamtpolarität des Extraktionsmediums einerseits und des Solubilisierungsvermögens des Tensides (Netzmittel) andererseits neben den bekannten Boswelliasäuren auch weitere möglicherweise pharmakologisch aktive Inhaltsstoffe extrahiert werden können.

Darüber hinaus können die Inhaltsstoffe des erfindungsgemäß erhaltenen Weihrauchextraktes, falls möglich, auch in physiologisch annehmbare Salze oder physiologisch annehmbare Derivate überführt werden. Physiologisch annehmbare Salze der Boswelliasäure und anderen sauren Verbindungen sind beispielsweise bevorzugt Natrium-, Kalium-, Ammonium- und Calciumsalze. Bevorzugte Derivate der Boswelliasäure sind C₁-C₈-Alkylester, bei denen die Carboxylgruppe der Boswelliasäure mit einem entsprechenden Alkohol verestert wurde. Besonders bevorzugt sind Methylester, Ethylester, n-Propylester, Isopropylester, n-Butylester, Isobutylester und Tertiärbutylester. Es ist beispielsweise ebenfalls möglich, dass die Hydroxylgruppe der Boswelliasäure mit einer physiologisch verträglichen Carbonsäure verestert ist, bevorzugt mit einer C₁-C₂₀-, insbesondere mit einer C₁-C₈-Carbonsäure, insbesondere mit Ameisensäure oder Essigsäure.

Die Erfindung wird anhand der beigefügten Figuren näher erläutert. Die Figuren zeigen Folgendes:

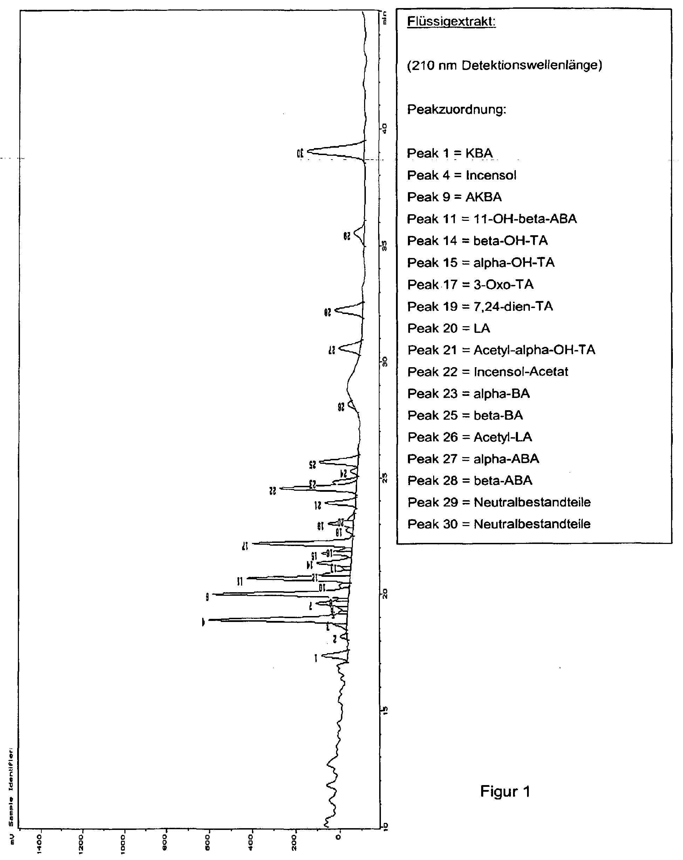

Figur 1: Qualitative Charakterisierung eines erfindungsgemäßen Weihrauchextrakts (Flüssigextrakt) mittels HPLC
Es wurden folgende Abkürzungen verwendet:

| **Name der Verbindung** | **Kürzel der Verbindung** |
|---|---|
| 11-Keto-ß-Boswelliasäure | KBA |
| 3-Oxo-Tirucallensäure | 3-Oxo-TA |
| 3ß-O-Acetyl-Boswelliasäure | ß-ABA |
| 3ß-OH-Tirucallensäure | ß-OH-TA |
| 3α-hydroxy-ß-Boswelliasäure | 3-α-OH-ß-BA |
| 3α-O-Acetyl-11-hydroxy-ß-Boswelliasäure | 11-OH-ß-ABA |
| 3α-O-Acetyl-11-Keto-ß-Boswelliasäure | AKBA |
| 3α-O-Acetyl-Boswelliasäure | α-ABA |
| 3α-O-Acetyl-Lupansäure | Acetyl-LA |
| 3α-O-Acetyl-Tirucallensäure | Acetyl-TA |
| 3α-OH-7,24-dien-Tirucallensäure | 7,24-dien-TA |
| 3α-OH-Tirucallensäure | α-OH-TA |
| Incensol | Incensol |
| Incensol-Acetat | Incensol-Acetat |
| Lupansäure | LA |
| ß-Boswelliasäure | ß-BA |
| α-Boswelliasäure | α-BA |

Figur 2: Qualitative Charakterisierung eines erfindungsgemäßen Weihrauchextrakts (Trockenextrakt) mittels HPLC
Figur 3: Vergleich eines erfindungsgemäß hergestellten Weihrauchextrakts (Figur 3a) mit Weihrauchextrakten, die nach dem Stand der Technik hergestellt wurden (Figuren 3b, c)
Figur 4: Pharmakologische Aktivität einzelner Fraktionen und des Gesamt-Extraktes (2 Chargen) bzgl. mPGES-1-Hemmung (zellfrei)
Figur 5: Pharmakologische Aktivität ausgewählter Fraktionen gegenüber reinen Rohsäuren-Fraktionen aus Standard-Methoden (Soxhlet, Methanol, Ethanol, Diethylether) bzgl. mPGES-1-Hemmung (zellfrei)

Die Erfindung wird anhand der nachstehenden Beispiele näher erläutert.

### BEISPIELE

### Beispiel 1: Herstellung eines Extrakts aus Weihrauch

Zur Herstellung des Weihrauchextrakts wurde zunächst eine Extraktionslösung als Extraktionsmedium angesetzt. Dazu wurden in 800 g Wasser 75 g Glycerin, 25 g Propylenglycol, 1 g Polysorbat 80 sowie 50 g einer 90 % Ethanollösung gegeben.

Zu dieser Extraktionslösung wurden 400 g eines gekörnten Weihrauchs gegeben. Danach wurde die Mischung kräftig durchgerührt, 24 Stunden bei Raumtemperatur stehen gelassen, abermals kräftig durchgerührt und anschließend filtriert. Die Ausbeute an Extrakt betrug 700 g.

Das so gewonnene Extrakt wurde auf seine Zusammensetzung sowie auf seine pharmakologische Aktivität untersucht. Dabei wurden ein Flüssigextrakt sowie ein Trockenextrakt, die in an sich bekannter Weise hergestellt worden sind, untersucht.

### Beispiel 2: Untersuchung des Weihrauchextrakts mittels HPLC

Proben des in Beispiel 1 erhaltenen Extrakts (2 Proben für Flüssigextrakte und 2 Proben für Trockenextrakte) wurden unter konstanten Bedingungen mit einer etablierten und verifizierten HPLC-Methode vermessen und die Signallagen qualitativ bei einer Wellenlänge von 210 nm verglichen (Retentionszeiten und relative Peakflächen). Zusätzlich wurden die Gehalte der bekannten Inhaltsstoffe Keto-Boswelliasäure (KBA) sowie Acetylketo-Boswelliasäure (AKBA) quantitativ angegeben (Peakflächenintegration bei 254 nm).

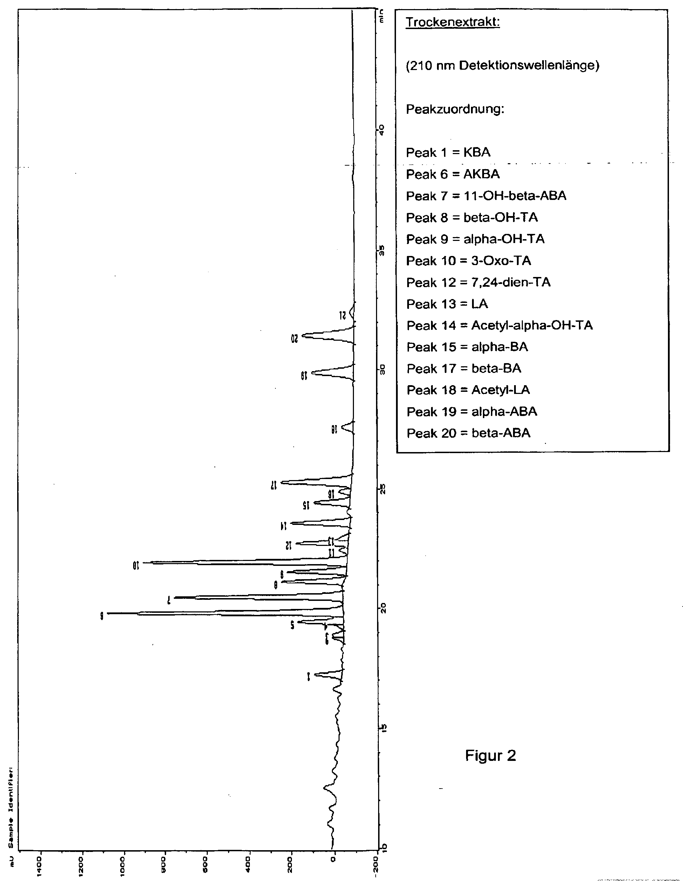

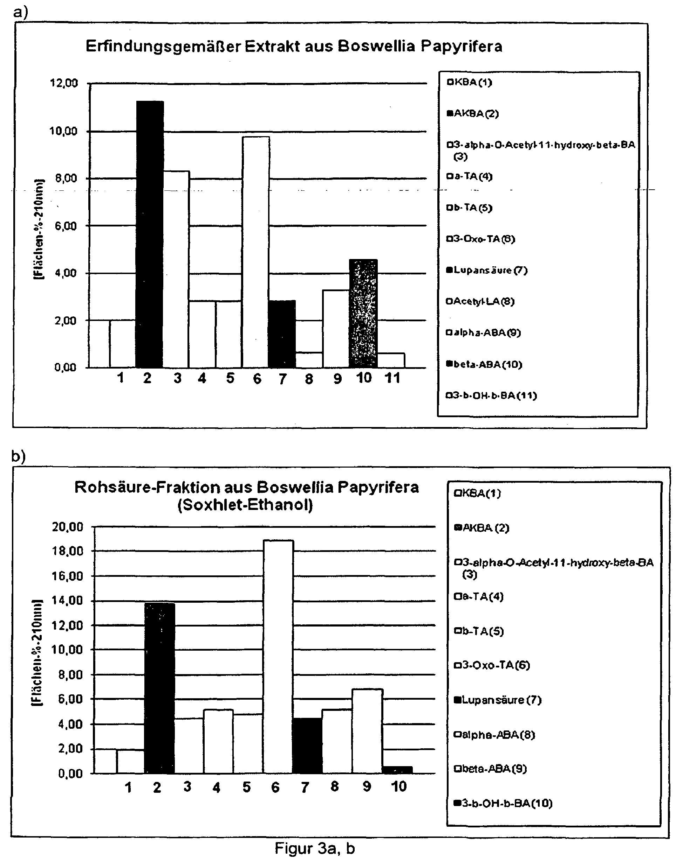

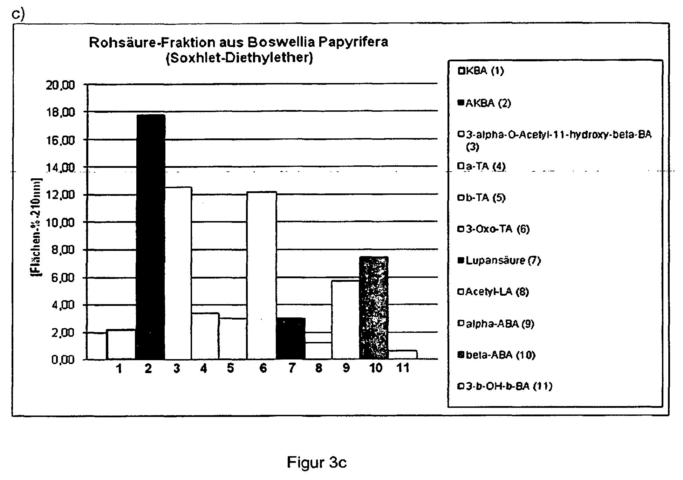

Die Ergebnisse sind in den Figuren 1 und 2 dargestellt. Die Figuren 3a, b, c zeigen einen Vergleich des erfindungsgemäßen Extrakts aus Boswellia papyrifera (3a) verglichen mit Extrakten, die nach Verfahren aus dem Stand der Technik hergestellt wurden (3b, c).

Die Extrakte wurden ferner quantitativ charakterisiert

Die Gehalte wurden in Prozent angegeben (siehe Tabelle). Die Werte korrelieren gut mit bisher für *Boswellia Papyrifera* ermittelten KBA- und AKBA-Gehaltsangaben. Es wurde immer mit einer Probenkonzentration von 5 mg/mL (Injektionskonzentration) gearbeitet. Die mittels externer Kalibrierung gewonnenen Gehalte wurden dann in Gewichtsprozent umgerechnet.

Zum Verständnis: Ein KBA-Gehalt von 0,6 % entspräche 6 mg in einem Gramm Probe des Extraktes. Ein AKBA-Gehalt von 4,0 % entspräche 40 mg in einem Gramm Probe des gleichen Extraktes.

**Tabelle 1**

| **rockenextrakte: 254 nm** | | | | |
|---|---|---|---|---|
| **Lauf-Nr.** | **KBA [Area]** | **Gehalt KBA [mg/mL]** | **AKBA [Area]** | **Gehalt AKBA [mg/mL]** |
| Inj. 24 | 2465666 | 0,04 | 18230882 | 0,40 |
| Inj. 30 | 1804960 | 0,03 | 13132056 | 0,29 |
| | **Gew.-% [g/g in %]** | **0,6 - 0,8%** | | **5,8 - 8,0%** |

**Tabelle 2**

| **lüssigextrakte: 254 nm** | | | | |
|---|---|---|---|---|
| **Lauf-Nr.** | **KBA [Area]** | **Gehalt KBA [mg/mL]** | **IARBA [Area]** | **Gehalt AKBA [mg/mL]** |
| Inj. 26 | 1446726 | 0,03 | 9373315 | 0,20 |
| Inj. 27 | 1275483 | 0,02 | 8563060 | 0, 19 |
| | **Gew.-% [g/g in %]** | **0,4 - 0,6%** | | **3,8 - 4,0%** |

Die Auswertung der vorstehenden Experimente ergibt folgende Ergebnisse:
a) Die Reproduzierbarkeit der im Weihrauchextrakt relevanten Inhaltsstoffe durch das beschriebene Extraktionsverfahren ist gegeben.
b) Das beschriebene Extraktionsverfahren liefert eine Mischung von Rohsäuren und Neutralbestandteilen.
c) Im Falle der qualitativen Auswertung (Vergleich der Retentionszeiten, Peakhöhen und Peakflächen bei 210 nm) der bisherigen Proben kann sowohl bei den Flüssig- als auch bei den Trockenextrakten jeweils von einem typischen "Fingerprint-Chromatogramm" gesprochen werden, das einen Flüssig- bzw. Trockenextrakt von *Boswellia Papyrifera* nach der hier beschriebenen Extraktionsmethode von anderen Extrakten dieses Weihrauchharzes unterscheidet.
d) Im Trockenextrakt wurden für KBA Gehalte von 0,6 - 0,8% (Gewichtsprozent; g/g) und für AKBA Gehalte von 5,8 - 8,0% (Gewichtsprozent; g/g) gefunden.
e) Im Flüssigextrakt wurden für KBA Gehalte von 0,4 - 0,6% (Gewichtsprozent; g/g) und für AKBA Gehalte von 3,8 - 4,0% (Gewichtsprozent; g/g) gefunden.
f) Die Gehaltswerte für KBA und AKBA sind in den Trockenextrakten größer, was sich dadurch erklären lässt, dass in den Flüssigextrakten ein erhöhter Anteil an Neutralbestandteilen vorliegt.
g) Auch das Verhältnis der Gehalte AKBA zu KBA (Verhältnis ca. 10:1; AKBA:KBA) entspricht typischen Werten von *Boswellia Papyrifera* und liegt für die beschriebene Extraktionsmethode sowohl bei den Flüssig- als auch den Trockenextrakten innerhalb akzeptabler Schwankungsgrenzen.

### Beispiel 3: mPGES-1-assay (zellfrei)

Im vorliegenden Beispiel wurde die pharmakologische Aktivität des Weihrauchextrakts gemäß Beispiel 1 mit Weihrauchextrakten, die nach dem Stand der Technik hergestellt wurden, untersucht.

Arachidonsäure (AA) ist vor allem in der Zellmembran lokalisiert. Sie wird durch mehrere Schritte enzymatisch abgebaut.

Im ersten Schritt wird die AA durch die zytosolische Phospholipase A2 (cPLA2) freigesetzt. Durch die Cyclooxygenase 1 und 2 wird die AA unter anderem weiterverarbeitet zu Prostaglandin G₂, welches dann auch von COX-1 und COX-2 zu Prostaglandin H₂ weiterverarbeitet wird.

PGH₂ wird von verschiedenen Synthasen abgebaut, darunter die mikrosomale Prostaglandin Synthase El (mPGES-1).Durch sie entsteht das Prostaglandin E₂.

COX-2 und mPGES-1 spielen eine wichtige Rolle bei entzündlichen Vorgängen.

### Mikrosomenaufreinigung

A549-Zellen (Lungenkarzinomzellen), die in DMEM (Dulbecco's Modified Eagle Medium) + FCS (Fetales Kälberserum) (10%)+ P/S (Penicillin/Streptornycin) kultiviert sind, werden für 72 h mit Interleukin-1β stimuliert. Nach 72 h erfolgt dann die Aufreinigung der Mikrosomen, diese werden dann für den mPGES-1-assay verwendet.

### Assay

Die Mikrosomen werden in einem Puffer auf einer 96-well-plate vorgelegt. Die zu untersuchenden Substanzen werden darin 15 min auf Eis vorinkubiert.

Danach erfolgt die einminütige Inkubation mit PGH₂ (20µM), die dann mit einer Stopplösung abgestoppt wird. Hierauf erfolgt die Festphasenextraktion woran dann im Anschluss die Auswertung an der HPLC erfolgt.

Man lässt sowohl eine Lösungsmittel-Kontrolle (100 %) mitlaufen als auch einen 0 Wert (spontane PGE₂-Bildung). Anhand dessen kann man dann eine prozentuale Aussage über die Restaktivität des Enzyms machen. Als zusätzliche Kontrolle dient der bekannte Inhibitor MK886 (IC50= 2,4 µM).

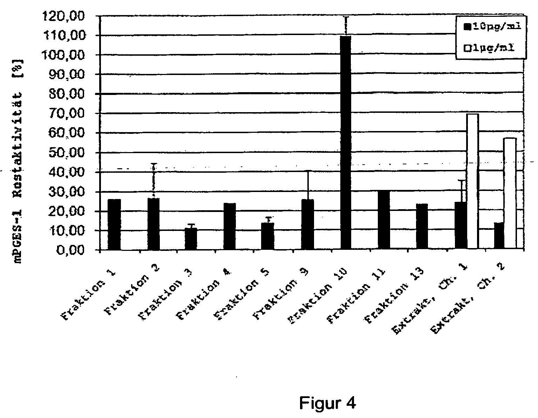

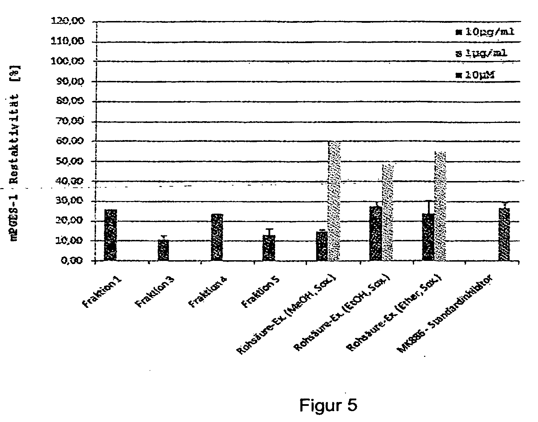

Die Ergebnisse sind in den Figuren 4 oder 5 dargestellt.

Zusammenfassend ist festzustellen, dass ein nach dem erfindungsgemäßen Verfahren hergestellter Extrakt folgende Vorteile zeigt:
1. Definierte Zusammensetzung
   a) der Harzsäure (vgl. Figuren 1, 2, 3)
   b) enthaltend Harzsäuren und weitere pharmakologisch aktive Substanzen (Neutralbestandteile, ätherische Öle)
2. Reproduzierbare Qualität
3. Nachgewiesene Wirkung in relevanten Test-Assays (AK Werz)
4. Erhältlich als Flüssigextrakt oder Trockenextrakt
5. Direkt zur innerlichen oder äußerlichen Anwendung einsetzbar
6. Mikrobiell und galenisch stabil (→ Lagerung bis 2 Jahre möglich)
7. Auftrennbar in verschiedene pharmakologisch aktive Fraktionen
8. Das Verfahren ist auf alle Boswellia-Spezies, bevorzugt Boswellia papyrifera, anwendbar.

## Patentansprüche

1. Verfahren zur Extraktion von pflanzlichen Harzen, die pharmakologisch aktive Inhaltsstoffe enthalten, **dadurch gekennzeichnet, dass** man
a) ein pflanzliches Rohharz aus einer Harz enthaltenden Pflanze bereitstellt,
b) das Rohharz mit einem Extraktionsmedium umfassend einen Wasseranteil von 50 bis 90 Gew.-%, ein oder mehrere hydrophile Lösungsmittel und ein oder mehrere Tensid(e) bei Raumtemperatur extrahiert, und
c) das so erhaltene Extraktionsprodukt in an sich bekannter Weise isoliert und gegebenenfalls weiter verarbeitet.

2. Verfahren nach Anspruch 1, **dadurch gekennzeichnet, dass** das pflanzliche Harz Weihrauchharz ist.

3. Verfahren nach Anspruch 1 oder 2, **dadurch gekennzeichnet, dass** das hydrophile Lösungsmittel aus ein- oder mehrwertigen Alkoholen niedriger Kettenlänge oder Gemischen davon ausgewählt ist.

4. Verfahren nach einem der Ansprüche 1 bis 3, **dadurch gekennzeichnet, dass** das Tensid aus O/W-Tensiden oder Kombinationen davon ausgewählt ist.

5. Verfahren nach einem der Ansprüche 1 bis 4, **dadurch gekennzeichnet, dass** der ein- oder mehrwertige Alkohol ausgewählt ist aus Methanol, Ethanol, Glycerin, Ethylenglykol, Propylenglykol, Butylenglykol oder Pexitylenglykol.

6. Verfahren nach einem der Ansprüche 1 bis 5, **dadurch gekennzeichnet, dass** das O/W-Tensid Polysorbat 60 oder Polysorbat 80 ist.

7. Verfahren nach einem der Ansprüche 1 bis 6, **dadurch gekennzeichnet, dass** das Extraktionsmedium 0,5 bis 50 Gew.-% Ethanol enthält.

8. Verfahren nach einem der Ansprüche 1 bis 7, **dadurch gekennzeichnet, dass** das Extraktionsmedium 1 bis 20 Gew.-% Glycerin enthält.

9. Verfahren nach einem der Ansprüche 1 bis 8, **dadurch gekennzeichnet, dass** das Extraktionsmedium 0,3 bis 10 Gew.-% Propylenglykol enthält.

10. Verfahren nach einem der Ansprüche 1 bis 9, **dadurch gekennzeichnet, dass** das Extraktionsmedium 0,01 bis 3 Gew.-% O/W-Tensid enthält.

11. Extraktionsprodukt eines pflanzlichen Harzes, erhalten nach einem Verfahren der Ansprüche 1 bis 10.

12. Extraktionsprodukt nach Anspruch 11 zur Verwendung als Arzneimittel, Medizinprodukt, funktionelles Lebensmittel, Nahrungsergänzungsmittel oder Kosmetikum.

13. Extraktionsprodukt, erhalten nach einem der Ansprüche 2 bis 10 zur Verwendung zur Behandlung von akuten und chronischen entzündlichen Erkrankungen, insbesondere von Erkrankungen des rheumatischen Formenkreises, Erkrankungen des Bewegungsapparates, entzündlichen Haut- und Darmerkrankungen, schmerzassoziierten Erkrankungen oder Tumorerkrankungen.

## Claims

1. A method for extraction of plant resins containing pharmacologically active constituents, **characterized by**
a) providing a plant crude resin from a resin-containing plant,
b) extracting the crude resin with an extraction medium, comprising a water content of 50 to 90 wt.%, one or more hydrophilic solvents and one or more detergent(s) at room temperature, and
c) isolating said thus obtained extraction product in an essentially known manner, and optionally further processing.

2. The method according to claim 1, **characterized in that** the plant resin is frankincense resin.

3. The method according to claim 1 or 2, **characterized in that** the hydrophilic solvent is selected from monohydric or polyhydric short-chain alcohols or mixtures thereof.

4. The method according to any one of claims 1 to 3, **characterized in that** the detergent is selected from O/W detergents or combinations thereof.

5. The method according to any one of claims 1 to 4, **characterized in that** the monohydric or polyhydric alcohol is selected from methanol, ethanol, glycerol, ethylene glycol, propylene glycol, butylene glycol or pentylene glycol.

6. The method according to any one of claims 1 to 5, **characterized in that** the O/W detergent is polysorbate 60 or polysorbate 80.

7. The method according to any one of claims 1 to 6, **characterized in that** the extraction medium contains 0.5 to 50 wt.% ethanol.

8. The method according to any one of claims 1 to 7, **characterized in that** the extraction medium contains 1 to 20 wit. % glycerol.

9. The method according to any one of claims 1 to 8, **characterized in that** the extraction medium contains 0.3 to 10 wt.% propylene glycol.

10. The method according to any one of claims 1 to 9, **characterized in that** the extraction medium contains 0.01 to 3 wt.% O/W detergent.

11. An extraction product of a plant resin obtained by any of the methods according to claims 1 to 10.

12. The extraction product according to claim 11 for use as drug, medicinal product, functional food, food supplement or cosmetic.

13. The extraction product obtained according to any one of claims 2 to 10 for use in the treatment of acute and chronic inflammatory diseases, in particular, rheumatic diseases, diseases of the locomotor system, inflammatory skin and bowel diseases, pain-associated diseases or tumor diseases.

## Revendications

1. Procédé d'extraction de résines végétales qui contiennent des constituants pharmacologiquement actifs, **caractérisé en ce que**
a) on fournit une résine végétale brute à partir d'une plante contenant une résine,
b) on extrait la résine brute avec un milieu d'extraction, comprenant un pourcentage d'eau de 50 à 90% en poids, un ou plusieurs tensioactifs, à température ambiante, et
c) on isole d'une manière connue le produit d'extraction ainsi obtenu, et éventuellement on le soumet à une post-transformation.

2. Procédé selon la revendication 1, **caractérisé en ce que** la résine végétale est la résine d'encens.

3. Procédé selon la revendication 1 ou 2, **caractérisé en ce que** le solvant hydrophile est choisi parmi les alcools monovalents ou polyvalents à longueur de chaîne courte ou des mélanges de ceux-ci.

4. Procédé selon l'une quelconque des revendications 1 à 3, **caractérisé en ce que** le tensioactif est choisi parmi les tensioactifs E/H ou des combinaisons de ceux-ci.

5. Procédé selon l'une quelconque des revendications 1 à 4, **caractérisé en ce que** l'alcool monovalent ou polyvalent est choisi parmi le méthanol, l'éthanol, le glycérol, l'éthylèneglycol, le propylèneglycol, le butylèneglycol ou le pentylèneglycol.

6. Procédé selon l'une quelconque des revendications 1 à 5, **caractérisé en ce que** le tensioactif E/H est le polysorbate 60 ou le polysorbate 80.

7. Procédé selon l'une quelconque des revendications 1 à 6, **caractérisé en ce que** le milieu d'extraction contient de 0,5 à 50% en poids d'éthanol.

8. Procédé selon l'une quelconque des revendications 1 à 7, **caractérisé en ce que** le milieu d'extraction contient de 1 à 20% en poids de glycérol.

9. Procédé selon l'une quelconque des revendications 1 à 8, **caractérisé en ce que** le milieu d'extraction contient de 0,3 à 10% en poids de propylèneglycol.

10. Procédé selon l'une quelconque des revendications 1 à 9, **caractérisé en ce que** le milieu d'extraction contient de 0,01 à 3% en poids de tensioactif E/H.

11. Produit d'extraction d'une résine végétale obtenu par un procédé selon les revendications 1 à 10.

12. Produit d'extraction selon la revendication 11 pour une utilisation comme médicament, produit médicinale, aliment fonctionnel, supplément alimentaire ou produit cosmétique.

13. Produit d'extraction, obtenu selon l'une quelconque des revendications 2 à 10 pour l'utilisation pour le traitement des maladies inflammatoires aiguës et chroniques en particulier des maladies rhumatismales, des maladies de l'appareil locomoteur, des maladies inflammatoires de la peau et de l'intestin, des maladies liées aux douleurs ou des maladies tumorales.
